# EUROPEAN PATENT SPECIFICATION

(11) **EP 2 939 616 B1**
(45) Date of publication and mention of the grant of the patent: **31.10.2018**
(21) Application number: 14200407.6
(22) Date of filing: 29.12.2014
(51) Int. Cl.: A61B 17/32, A61B 18/14

(54) **Electrosurgical instruments including end-effector assembly configured to provide mechanical cutting action on tissue**
Elektrochirurgiegeräte mit Endeffektoranordnung zum mechanischen Schneiden von Gewebe
Instruments électrochirurgicaux comprenant un ensemble effecteur terminal permettant d'exercer une action de coupe mécanique sur le tissu

(30) Priority: 02.05.2014 US 201461987534 P; 18.12.2014 US 201414574851
(43) Date of publication of application: 04.11.2015
(73) Proprietor: Covidien LP, Mansfield, MA 02048 (US)
(72) Inventor: Allen IV, James D., Broomflied, Colorado 80020 (US)
(74) Representative: Soames, Candida Jane

(56) References cited:
- EP-A1- 1 875 875
- WO-A1-2013/141218
- WO-A1-2015/077119
- WO-A2-2014/078548
- US-A1- 2010 063 500

## Description

### BACKGROUND

### 1. Technical Field

The present disclosure relates to electrosurgical devices. More particularly, the present disclosure relates to electrosurgical instruments having an end-effector assembly coupled to a vibration mechanism and configured to provide a mechanical cutting action on tissue, electrosurgical systems including the same, and exemplary methods of sealing and cutting tissue using the same.

### 2. Discussion of Related Art

Electrosurgery involves the application of thermal and/or electrical energy to cut, dissect, ablate, coagulate, cauterize, seal, or otherwise treat tissue during a surgical procedure. Electrosurgery is typically performed using an electrosurgical generator operable to output energy to an electrosurgical instrument adapted to transmit energy to a tissue site to be treated. Electrosurgical instruments, such as electrosurgical forceps, have come into widespread and accepted use in both open and minimally-invasive surgical procedures. By utilizing an electrosurgical forceps, a surgeon can cauterize, coagulate, desiccate and/or seal tissue and/or simply reduce or slow bleeding by controlling the intensity, frequency and duration of the electrosurgical energy applied through the jaw members to the tissue.

Some surgical instruments utilize ultrasonic vibrations to effectuate treatment of tissue. When transmitted at suitable energy levels, ultrasonic vibrations may be used to coagulate, cauterize, fuse, seal, cut, desiccate, and/or fulgurate tissue, and various levels of hemostasis can be achieved. EP 1875875 A1 and WO 2013/141218 A1 disclose electrosurgical instruments which can apply both electrosurgical energy and mechanical vibration to treat tissue.

Electrosurgical devices have been manufactured with two or more separate switches and corresponding buttons that transition the device from a first power level to a second power level on a first electrical mode to a second electrical mode. As such, two-switch devices require the operator to differentiate between the low-power switch and the higher-power switch. This requirement for differentiation can lead to the operator having to look at a portion of the device that is not easily visible from the operator's current view, and, during surgery, can lead to adverse and unwanted effects if the wrong button is hit.

### SUMMARY

Electrosurgical instruments in accordance with this disclosure can apply both electrosurgical energy and mechanical vibration to treat tissue. The invention provides an electrosurgical instrument according to claim 1. Further embodiments of the invention are provided in the dependent claims. The present electrosurgical instruments may be employed in an electrosurgical system to perform electrosurgical procedures.

### BRIEF DESCRIPTION OF THE DRAWINGS

The above and other aspects and features of the presently-disclosed electrosurgical instruments having an end-effector assembly coupled to a vibration mechanism and configured to provide a mechanical cutting action on tissue, electrosurgical systems including the same, and methods of sealing and cutting tissue using the same, will become apparent to those of ordinary skill in the art when descriptions of various embodiments thereof are read with reference to the accompanying drawings, of which:
FIG. 1 is a left, perspective view of an electrosurgical instrument in accordance with an embodiment of the present disclosure;
FIG. 2 is an internal, side view of the electrosurgical instrument of FIG. 1, shown with parts separated, in accordance with an embodiment of the present disclosure;
FIG. 3 is an enlarged, perspective partial view showing the end-effector assembly of the electrosurgical instrument of FIG. 1 in accordance with an embodiment of the present disclosure;
FIG. 4A is an enlarged, side view of a portion of a jaw member, showing an embodiment of a protruding element formed as a serrated strip in accordance with the present disclosure;
FIG. 4B is an enlarged, end view of the jaw member of FIG. 4A;
FIG. 5 is an enlarged, side partial view showing the end-effector assembly of FIG. 3;
FIG. 6 is an enlarged, side partial view of another embodiment of an end-effector assembly in accordance with the present disclosure;
FIG. 7 is an enlarged, perspective partial view of another embodiment of an end-effector assembly in accordance with the present disclosure;
FIG. 8 is an enlarged, perspective partial view of the end-effector assembly of FIG. 1 showing the first and second jaw members thereof in a closed configuration in accordance with an embodiment of the present disclosure;
FIG. 9 is a schematic block diagram of an electrosurgical system in accordance with an embodiment of the present disclosure;
FIG. 10 is a schematic view of the end-effector assembly of the electrosurgical instrument of FIG. 1 as connected to a piezoelectric oscillation mechanism via a vibration coupler in accordance with an embodiment of the present disclosure;
FIG. 11 is a schematic view of the end-effector assembly of the electrosurgical instrument of FIG. 1 as connected to an eccentric cam oscillation mechanism via a vibration coupler in accordance with an embodiment of the present disclosure;
FIG. 12 is a schematic view of the end-effector assembly of the electrosurgical instrument of FIG. 1 as connected to a counterweight oscillation mechanism via a vibration coupler in accordance with an embodiment of the present disclosure;
FIG. 13 is a schematic view of the end-effector assembly of the electrosurgical instrument of FIG. 1 as connected to a voice coil oscillation mechanism via a vibration coupler in accordance with an embodiment of the present disclosure;
FIG. 14 is a flowchart illustrating a method of treating tissue in accordance with an embodiment of the present disclosure; and
FIG. 15 is a flowchart illustrating a method of treating tissue in accordance with another embodiment of the present disclosure.

### DETAILED DESCRIPTION

Hereinafter, embodiments of an electrosurgical instrument including an end-effector assembly coupled to a vibration mechanism and configured to provide a mechanical cutting action on tissue, electrosurgical systems including the same, and methods of sealing and cutting tissue using the same of the present disclosure are described with reference to the accompanying drawings. Like reference numerals may refer to similar or identical elements throughout the description of the figures. As shown in the drawings and as used in this description, and as is traditional when referring to relative positioning on an object, the term "proximal" refers to that portion of the apparatus, or component thereof, closer to the user and the term "distal" refers to that portion of the apparatus, or component thereof, farther from the user.

This description may use the phrases "in an embodiment," "in embodiments," "in some embodiments," or "in other embodiments," which may each refer to one or more of the same or different embodiments in accordance with the present disclosure.

Vessel sealing or tissue sealing utilizes a combination of radiofrequency (RF) energy, pressure and gap control to effectively seal or fuse tissue between two opposing jaw members or electrically-conductive sealing plates thereof. Tissue pressures within a working range of about 3 kg/cm² to about 16 kg/cm² and, advantageously, within a working range of 7 kg/cm² to 13 kg/cm² have been shown to be effective for sealing arteries and vascular bundles. Vessel or tissue sealing is more than "cauterization" which may be defined as the use of heat to destroy tissue (also called "diathermy" or "electrodiathermy"), and vessel sealing is more than "coagulation" which may be defined as a process of desiccating tissue wherein the tissue cells are ruptured and dried. As it is used in this description, "vessel sealing" generally refers to the process of liquefying the collagen, elastin and ground substances in the tissue so that it reforms into a fused mass with significantly-reduced demarcation between the opposing tissue structures.

As used herein, the terms "power source" and "power supply" refer to any source of electrical power, e.g., electrical outlet, a/c generator, battery or battery pack, etc. As it is used in this description, "electrically conductive," or simply "conductive," generally refers to materials that are capable of electrical conductivity, including, without limitation, materials that are highly conductive, e.g., metals and alloys, or materials that are semi-conductive, e.g., semi-conducting materials and composites. As it is used in this description, "transmission line" generally refers to any transmission medium that can be used for the propagation of signals from one point to another. As it is used in this description, "switch" or "switches" generally refers to any electrical actuators, mechanical actuators, electro-mechanical actuators (rotatable actuators, pivotable actuators, toggle-like actuators, buttons, etc.), optical actuators, or any suitable device that generally fulfills the purpose of connecting and disconnecting electronic devices, or component thereof, instruments, equipment, transmission line or connections and appurtenances thereto, or software.

Various embodiments of the present disclosure provide an electrosurgical instrument with an end-effector assembly coupled to a vibration mechanism and configured to provide a mechanical cutting action on tissue. Various embodiments of the present disclosure provide electrosurgical instruments configured to provide monopolar electrosurgical energy and/or bipolar electrosurgical energy, which may be suitable for sealing, cauterizing, coagulating, desiccating, and/or cutting tissue, e.g., vessels and vascular tissue. Embodiments of the presently-disclosed electrosurgical instruments may be suitable for utilization in endoscopic surgical procedures and/or suitable for utilization in open surgical applications.

Embodiments of the presently-disclosed electrosurgical instruments may be implemented using a variety of types of energy, e.g., electrosurgical energy at radio frequencies (RF) and/or at other frequencies, optical, and/or thermal energy. Embodiments of the presently-disclosed electrosurgical instruments may be configured to be connectable to one or more energy sources, e.g., RF generators and/or self-contained power sources. Embodiments of the presently-disclosed electrosurgical instruments may be connected through a suitable bipolar cable and/or other transmission line to an electrosurgical generator and/or other suitable energy source.

Various embodiments of the present disclosure provide an electrosurgical system including an electrosurgical instrument having an end-effector assembly including opposing jaw members, wherein at least one of the jaw members is coupled to a vibration mechanism and configured to provide a mechanical cutting action on tissue with minimal heating of the oscillating jaw member. One or both of the jaw members of the presently-disclosed end-effector assemblies are provided with electrically-conductive elements and/or electrically non-conductive elements, which may be configured as a tissue-engaging surface, an elongated ribbon-like or strip-like member, a wire, a wire-like member, a rod-like member, or a deposited coating. Various embodiments of the presently-disclosed electrosurgical instruments are configured to oscillate one of the jaw members to cause mechanical friction on tissue to cause it to cut. Various embodiments of the presently-disclosed electrosurgical instrument and electrosurgical systems including the same may include any feature or combination of features of the end-effector assembly embodiments and vibration mechanisms disclosed herein.

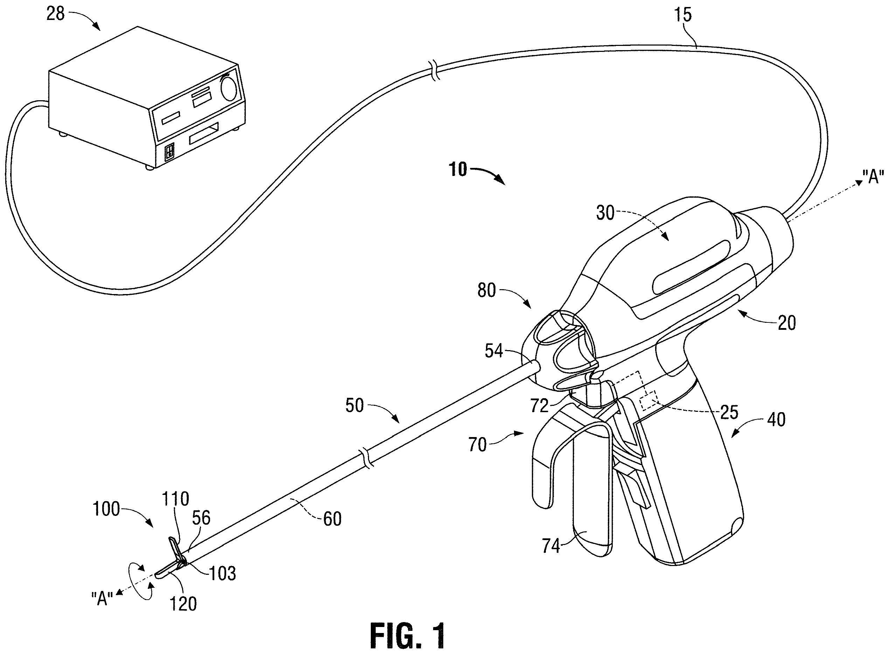

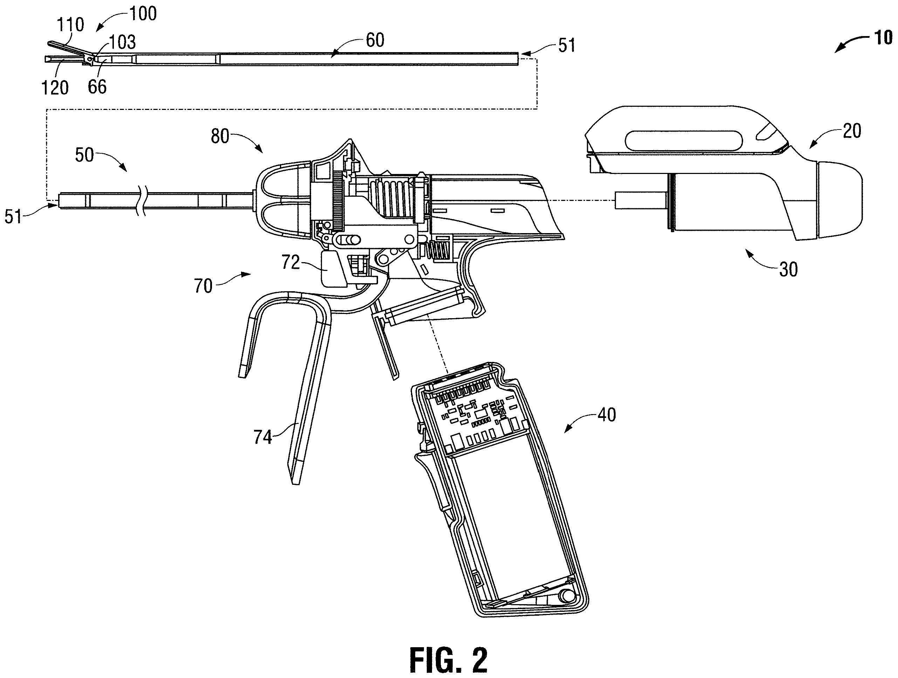

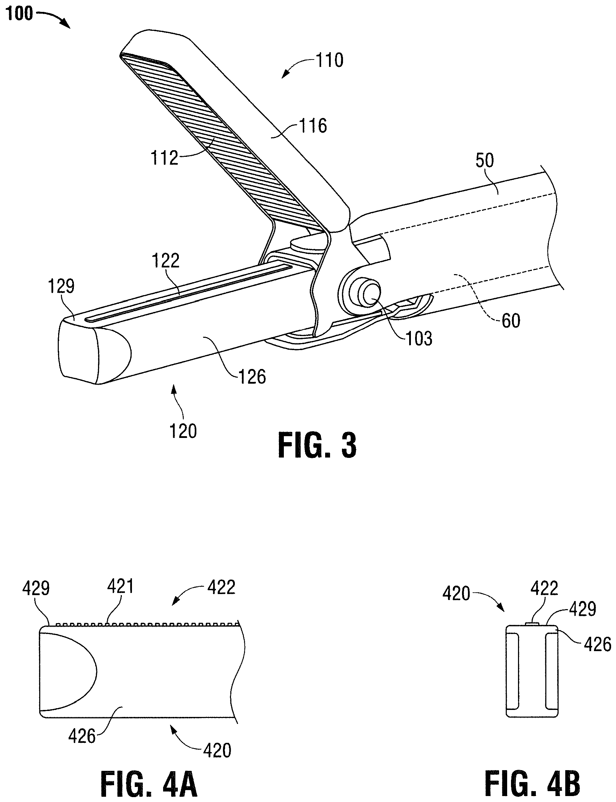

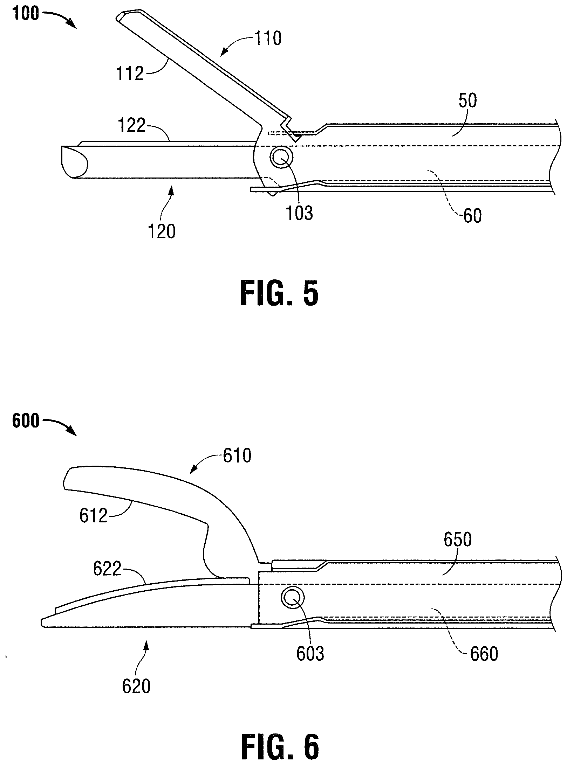

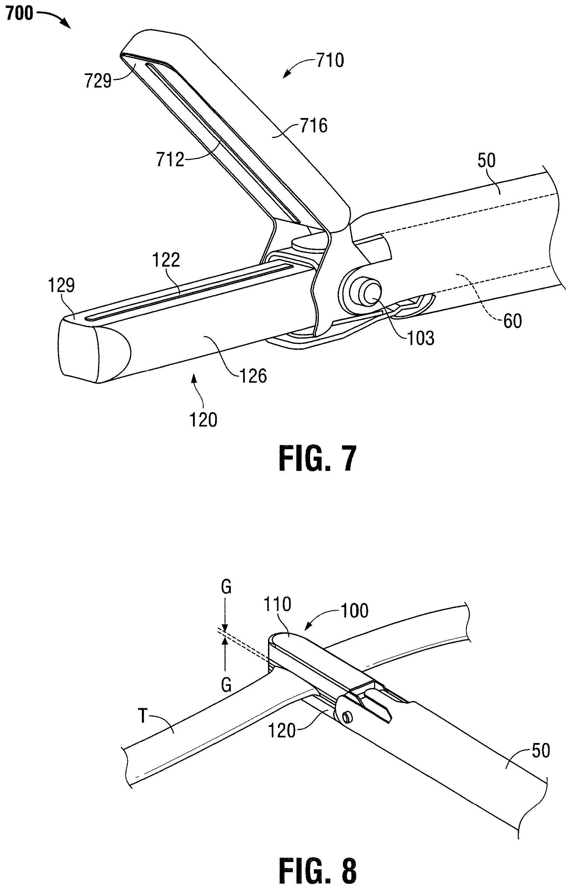

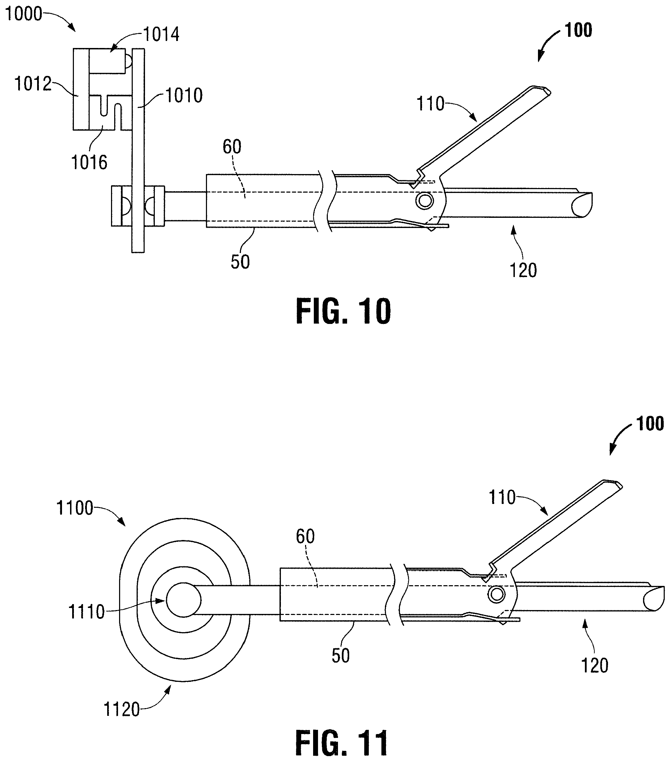

In FIGS. 1 and 2, an embodiment of an electrosurgical instrument 10 is shown for use with various surgical procedures and generally includes a housing 20, an oscillation mechanism 30 configured to generate a mechanical vibration in a vibration coupler 60, a rotation knob 80, a trigger assembly 70, and an end-effector assembly 100. An embodiment of the end-effector assembly 100 of FIGS. 1 and 2 is shown in more detail in FIGS. 3 and 5. It is to be understood, however, that other end-effector assembly embodiments (e.g., the end-effector assembly 600 shown in FIG. 6 and the end-effector assembly 700 shown in FIG. 7) may also be used. Examples of sources of mechanical vibrations that may be suitable for use as the oscillation mechanism 30 are shown in FIGS. 10 through 13. Electrosurgical instrument 10 may include additional, fewer, or different components than shown in FIGS. 1 and 2, depending upon a particular purpose or to achieve a desired result.

Electrosurgical instrument 10 includes an elongated shaft 50 having a distal end 56 configured to mechanically engage the end-effector assembly 100. End-effector assembly 100, which is described in more detail later in this disclosure, generally includes two jaw members 110 and 120 disposed in opposing relation relative to one another. Shaft 50, which may be at least partially disposable, extends from the housing 20 and defines a central lumen 51 (FIG. 2) therethrough. Shaft 50 supports movement of other components through the central lumen 51, e.g., to impart movement to the upper jaw member 110 and/or to impart vibration energy to the lower jaw member 120. The proximal end 54 of the shaft 50 is received within the housing 20 or is otherwise engaged to the housing 20, and connections relating thereto are disclosed in U.S. Patent No. 7,156,846 entitled "Vessel Sealer And Divider For Use With Small Trocars And Cannulas," U.S. Patent No. 7,597,693 entitled "Vessel Sealer And Divider For Use With Small Trocars And Cannulas" and U.S. Patent No. 7,771,425 entitled "Vessel Sealer And Divider Having A Variable Jaw Clamping Mechanism."

Although FIG. 1 depicts an electrosurgical forceps for use in connection with endoscopic surgical procedures, the teachings of the present disclosure may also apply to more traditional open surgical procedures. The electrosurgical instrument 10 is described in terms of an endoscopic instrument; however, it is contemplated that an open version of a forceps may also include the same or similar operating components and features as described below.

Rotation knob 80 is operably coupled to the shaft 50 and rotatable about a longitudinal axis "A - A" defined by the shaft 50. As depicted in FIG. 1, the end-effector assembly 100 is rotatable in either direction about the longitudinal axis "A - A" through rotation, either manually or otherwise, of the rotation knob 80. One or more components of the electrosurgical instrument 10, e.g., the housing 20, the rotation knob 80, the trigger assembly 70, and/or the end-effector assembly 100, may be adapted to mutually cooperate to grasp, seal and/or divide tissue, e.g., tubular vessels and vascular tissue (e.g., tissue "T" shown in FIG. 8).

In some embodiments, as shown in FIGS. 1, 2, 3 and 5, the end-effector assembly 100 is configured as a unilateral assembly that includes a stationary jaw member 120 mounted in fixed relation to the shaft 50 and a pivoting jaw member 110 movably mounted about a pivot pin 103 coupled to the fixed jaw member 120. Jaw members 110 and 120 may be curved at various angles to facilitate manipulation of tissue and/or to provide enhanced line-of-sight for accessing targeted tissues. End-effector assembly 100 may include one or more electrically-insulative elements to electrically isolate the pivoting jaw member 110 (also referred to herein as "first jaw member 110") from the stationary jaw member 120 (also referred to herein as "second jaw member 120") and/or to isolate both or one of the jaw members 110 and 120 from the shaft 50. Alternatively, the electrosurgical instrument 10 may include a bilateral assembly, i.e., both jaw members 110 and 120 move relative to one another.

Referring to FIG. 3, the first and second jaw members 110 and 120 include first and second structural support members 116 and 126, respectively. First and second structural support members 116 and 126 may be formed from any suitable material or combination of materials, e.g., metallic material, plastic and the like, and may be formed by any suitable process, e.g., machining, stamping, electrical discharge machining (EDM), forging, casting, injection molding, metal injection molding (MIM), and/or fineblanking. Examples of metallic material that may be suitable include aluminum and alloys thereof, plated brass, stainless steel, stainless steel alloys, beryllium copper, etc. In some embodiments, one or both of the first and second structural support members 116 and 126 may be formed from material having malleable or flexible properties or, alternatively, one or both of the first and second structural support members 116 and 126 may be formed from rigid material.

According to the present invention, the second jaw member 120 includes an electrically-conductive tissue-engaging surface 129 (also referred to herein as "conductive sealing surface 129") and a protruding element 122 extending therefrom. Protruding element 122, as shown in FIG. 3, is configured as an elongated strip extending along a length of the second jaw member 120, and formed of an electrically non-conductive material. In examples not part of the present invention, the protruding element 122 may be configured as an elongated ribbon-like member, a wire, a wire-like member, a rod-like member, or a deposited coating. In examples not part of the present invention, the protruding element 122 may be formed of an electrically-conductive material, e.g., metal, on the support member 126 (or the conductive sealing surface 129), e.g., using a deposition technique, stamping, etching, machining and/or any other suitable method that may be used to form an elongated strip-like conductor. In examples not part of the present invention, the protruding element 122 may be integrally formed with the conductive sealing surface 129. Protruding element 122 may be curved or straight, e.g., depending upon a particular surgical purpose.

According to the present invention, as shown for example in FIGS. 3 and 5, the first jaw member 110 includes an electrically-conductive tissue-engaging surface 112 that is configured as a tissue-sealing plate. Electrically-conductive tissue-engaging surface 112 (also referred to herein as "tissue-sealing plate 112") may be formed by stamping, overmolding, machining, and/or any other suitable method that may be used to form a sealing plate.

Structural support members 116 and 126 may be configured to support the tissue-sealing plate 112 and the conductive sealing surface 129, respectively, and may be manufactured from any suitable material, e.g., metal, plastic and the like. In some embodiments, the conductive sealing surface 129 may be a single face of the structural support member 126, and may include any suitable electrically-conductive material, e.g., metal. In other embodiments, the conductive sealing surface 129 may be a thin metal stamping or thin metal plating coupled to the structural support member 126 in a manner that electrically isolates the conductive sealing surface 129 from the structural support member 126. In some embodiments, the structural support member 126 is formed of an electrically and thermally insulative material, e.g., a temperature resistant plastic or a ceramic, overmolded onto the conductive sealing surface 129. Conductive sealing surface 129 may be configured to be electrically connectable to one pole of a bipolar energy source (e.g., electrosurgical power generating source 28 shown in FIG. 1).

In some embodiments, the electrosurgical instrument 10 is configured to allow the conductive sealing surface 129 to be electrically connectable to the monopolar active terminal of an electrosurgical generator. Some electrosurgical generators include two bipolar terminals and active and passive monopolar terminals, and it is contemplated that two different types of instruments, monopolar and bipolar, or a combination of both, can be connected to the generator simultaneously.

The second jaw member 120 may be configured as a rod that mechanically connects to an oscillation mechanism 30 (FIG. 1) that is configured to generate a mechanical vibration that causes the second jaw member 120 to oscillate along its axis at a pre-determined frequency and amplitude, wherein the conductive sealing surface 129 and the protruding element 122 oscillate one and the same with the second jaw member 120. Protruding element 122 may be configured as a hardened abrasive material, e.g., ceramic, titanium oxide or other metal oxides, glass, or other suitable material, and may include serrations 421 (FIG. 4A), e.g., to enhance abrasiveness.

In some embodiments, the electrosurgical instrument 10 is configured to allow the oscillation mechanism 30 to be energized and activated by the user. Electrosurgical instrument 10 may be configured to seal and cut via user control, e.g., first button stage seals tissue using bipolar energy, and second button stage activates oscillation to cut tissue while bipolar energy is still active.

In other embodiments, the electrosurgical instrument 10 is configured to allow the oscillation mechanism 30 to be energized automatically by the electrosurgical power generating source 28 when certain input criteria is met, such as RF energy activation time, tissue impedance, first jaw member 110 opening angle, force applied to tissue, and/or thickness of tissue. In some embodiments, the electrosurgical instrument 10 is configured to seal and cut automatically using bipolar energy and oscillation, wherein the electrosurgical power generating source 28 determines when to cut based on surgeon inputs and/or various sensor inputs.

In accordance with various embodiments of the present disclosure, when tissue "T" (FIG. 8) is grasped under pressure between the first jaw member 110 and the second jaw member 120, and when the oscillation mechanism (e.g., oscillation mechanism 1000, 1100, 1200, or 1300 shown in FIGS. 10 through 13, respectively) is activated, the protruding element 122 applies a concentrated pressure and oscillation to the tissue "T" so as to cut and divide it. The effectiveness and speed of the cut may be enhanced by the condition of the tissue "T" after bipolar sealing energy has been applied to the tissue "T" between tissue-sealing plate 112 and the conductive sealing surface 129. In general, the bipolar sealing energy heats the tissue "T" which causes components of the tissue "T" to meld together, desiccate, and/or break down. During the delivery of energy to tissue "T," moisture is driven out of the seal zone and the cellular structure of the tissue "T" is weakened, making it easier to cut with the reciprocation of the protruding element 122.

In some embodiments, the energy that seals the tissue may be delivered using monopolar energy, wherein the tissue-sealing plate 112 and the conductive sealing surface 129 are the same potential, and energy travels from the tissue-sealing plate 112 and the conductive sealing surface 129, through the patient, through a grounding pad, and back to the generator (e.g., similar to a surgical technique referred to as "buzzing the hemostat"). In some embodiments, the electrosurgical instrument 10 may be configured to apply monopolar energy through only one jaw member for making holes in tissue and/or coagulating when the jaw members are open or closed without tissue grasped therebetween.

In some embodiments, electrosurgical instrument 10 may be capable of switching between monopolar and bipolar modes depending on user inputs and/or automatic generator control.

According to the present invention, wherein the protruding element 122 is formed of an electrically non-conductive material, the protruding element 122 serves as jaw gap control in bipolar configurations, e.g., preventing the tissue-sealing plate 112 and the conductive sealing surface 129 from making an electrical connection therebetween. In examples not part of the present invention, in monopolar configurations, wherein the tissue-sealing plate 112 and the conductive sealing surface 129 do not need to be electrically isolated from one another because they are at the same electric potential, the protruding element 122 may be formed of an electrically-conductive material. In monopolar configurations, it may be advantageous (e.g., to increase cutting speed) to utilize an electrically-conductive protruding element 122 so as to concentrate monopolar energy to the cut region on the tissue while the second jaw member 120 oscillates.

In some embodiments, the protruding element 112 may have a thickness that varies (i.e., non-uniform) from a proximal end to a distal end thereof.. For example, the protruding element 122 may have a proximal end having a thickness that is slightly larger than a thickness at the distal end thereof, e.g., depending on a particular purpose. Protruding element 112, or portions thereof, may be formed as a serrated strip configuration, e.g., similar to the protruding element 422 shown in FIGS. 4A and 4B.

As shown in FIGS. 3 and 5, first jaw member 110 includes a tissue-engaging surface 112. According to the present invention, the tissue-engaging surface 112 is formed of an electrically-conductive material. In examples not part of the present invention, the tissue-engaging surface 112 may be formed of an electrically non-conductive material. As an alternative to, or in addition to, an electrically non-conductive tissue-engaging surface 112, the first structural support member 116, or portions thereof, may be formed of an electrically non-conductive material. End-effector assembly 100 may additionally, or alternatively, include electrically-insulative members and/or electrically-insulative, thermally non-degrading coatings configured to electrically isolate, at least in part, the tissue-sealing plate 112 and the conductive sealing surface 129 from the first and second structural support members 116 and 126, respectively.

Referring to FIGS. 1 and 2, electrosurgical instrument 10 includes a vibration coupler 60 configured to transmit vibrations from the oscillation mechanism 30 to the end-effector assembly 100. Second jaw member 120 receives at least a portion of a vibration coupler 60 therein. In some embodiments, the vibration coupler 60 is movably coupled to the proximal end of the second jaw member 120. In some embodiments, the vibration coupler 60 may be integrally formed with the proximal end of the second jaw member 120. Second jaw member 120 is configured to receive the mechanical vibration from the vibration coupler 60 and transmit the mechanical vibration to treat tissue positioned within the end-effector assembly 100, e.g., to provide a mechanical cutting action on tissue (e.g., tissue "T" shown in FIG. 8).

Trigger assembly 70 is operably coupled to the housing 20 and generally includes an activation switch 72 and a clamping trigger 74. Activation switch 72 is configured to facilitate the transmission of the energy from one or more energy sources, e.g., electrosurgical power generating source 28 and/or oscillation mechanism 30, to the end-effector assembly 100. In some embodiments, the clamping trigger 74 of the trigger assembly 70 is operatively connected to the shaft assembly 50 to impart movement to the first jaw member 110 from an unapproximated (open) configuration (FIG. 1), where the first and second jaw members 110 and 120 are disposed in spaced relation relative to one another, to a clamping or approximated (closed) configuration (FIG. 8), wherein the first and second jaw members 110 and 120 cooperate to engage and grasp tissue therebetween.

In some embodiments, when the activation switch 72 is actuated, the oscillation mechanism 30 is activated and applies energy, e.g., mechanical energy in the form of vibrations, to the vibration coupler 60. As discussed above, the mechanical vibration is transmitted from the oscillation mechanism 30 along the vibration coupler 60 to the end-effector assembly 100, e.g., to treat tissue (e.g., tissue "T" shown in FIG. 8) overlying the second jaw member 120 and/or grasped between the first and second jaw members 110 and 120.

Electrosurgical instrument 10 generally includes a controller 25. In some embodiments, as shown in FIG. 1, the controller 25 is formed integrally with the electrosurgical instrument 10. In other embodiments, the controller 25 may be provided as a separate component coupled to the instrument 10. Controller 25 may include any type of computing device, computational circuit, or any type of processor or processing circuit capable of executing a series of instructions that are stored in a memory. Controller 25 may be configured to control one or more operating parameters associated with the electrosurgical power generating source 28 based on one or more signals indicative of user input, such as generated by the activation switch 72 and/or one or more separate, user-actuatable buttons or switches. Examples of switch configurations that may be suitable for use with the electrosurgical instrument 10 include, but are not limited to, pushbutton, toggle, rocker, tactile, snap, rotary, slide and thumbwheel. In some embodiments, the instrument 10 may be selectively used in either a monopolar mode or a bipolar mode by engagement of the appropriate switch.

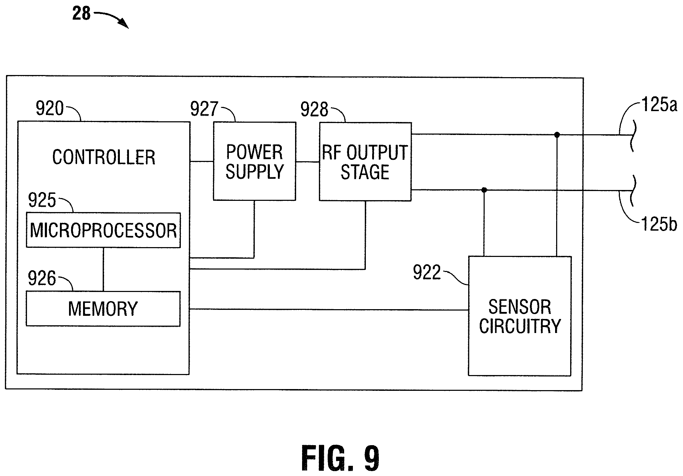

As an alternative to, or in addition to, the trigger assembly 70, electrosurgical instrument 10 may include voice input technology, which may include hardware and/or software, which may be incorporated into the instrument 10, or component thereof (e.g., controller 25), and/or incorporated into the controller 920 shown in FIG. 9, or a separate digital module connected to the controller 920. The voice input technology may include voice recognition, voice activation, voice rectification, and/or embedded speech. The user may be able to control the operation of the instrument 10 in whole or in part through voice commands, e.g., freeing one or both of the user's hands for operating other instruments. Voice or other audible output may also be used to provide the user with feedback.

In some embodiments, as shown in FIG. 1, activation switch 72 is operably coupled to the housing 20 and electrically connected (as indicated by the dashed lines in FIG. 1) to the controller 25. In some embodiments, activation switch 72 is configured as a two-stage switch wherein a first stage of the switch 72 effects a first operational mode of two or more operational modes and a second stage of the switch 72 effects a second operational mode that is different from the first operational mode. In some embodiments, in the first operational mode, energy is provided for sealing, but vibration of the second jaw 120 including the protruding element 122 is not activated. In some embodiments, in the second operational mode, vibration energy is imparted to the second jaw member 120.

Switch 72 may have a variable resistance such that the first stage occurs when a first depression force is applied to the switch 72, and the second stage occurs when a second depression force greater than the first depression force is applied to the switch 72. The first depression force and/or the second depression force may cause electrical contacts within the switch 72 to close, thereby completing a circuit between the end-effector assembly 100 and an energy source, e.g., electrosurgical power generating source 28. Of course, the description of closing electrical contacts in a circuit is, here, merely an example of switch operation. There are many alternative embodiments, some of which may include opening electrical contacts or processor-controlled power delivery that receives information from the switch 72 and directs a corresponding circuit reaction based on the information. In some embodiments, when a first depression force is applied to the switch 72, the electrosurgical instrument 10 transitions to the first operational mode, e.g., energy is provided for sealing, but vibration of the second jaw 120 including the protruding element 122 is not activated. In some embodiments, when a second depression force is applied to the switch 72, the electrosurgical instrument 10 transitions to the second operational mode, e.g., vibration energy is imparted to the second jaw member 120.

In some embodiments, as shown in FIG. 1, electrosurgical instrument 10 includes a transmission line 15, which may connect directly to the electrosurgical power generating source 28. Transmission line 15 may be formed from a suitable flexible, semi-rigid or rigid cable, and may be internally divided into one or more cable leads (e.g., leads 125a and 125b shown in FIG. 9) each of which transmits energy through its respective feed path to the end-effector assembly 100.

Electrosurgical power generating source 28 may be any generator suitable for use with electrosurgical devices, and may be configured to operate in a variety of modes such as monopolar and bipolar cutting, coagulation, and other modes. Examples of electrosurgical generators that may be suitable for use as a source of electrosurgical energy include generators sold by Covidien Surgical Solutions of Boulder, CO, e.g., Ligasure™ generator, FORCE EZ™ electrosurgical generator, FORCE FX™ electrosurgical generator, FORCE TRIAD™ electrosurgical generator, or other generators which may perform different or enhanced functions. An embodiment of a standalone electrosurgical generator, such as the electrosurgical power generating source 28 of FIG. 1, in accordance with the present disclosure, is shown in more detail in FIG. 9. It will be understood, however, that other standalone electrosurgical generator embodiments may also be used. In some embodiments, a distal portion of the transmission line 15 may be disposed within the housing 20.

Electrosurgical instrument 10 may alternatively be configured as a battery-powered wireless instrument. In some embodiments, electrosurgical instrument 10 is powered by a self-contained power source 40 (FIG. 1) when the power source 40 is operably connected to the instrument 10. Self-contained power source 40 may include any combination of battery cells, a battery pack, fuel cell and/or high-energy capacitor for use to provide power to the instrument 10.

FIGS. 4A and 4B show a portion of a jaw member 420 that includes a structural support member 426 and a protruding element 422 extending from a conductive sealing surface 429 associated with the structural support member 426. Protruding element 422 is formed as a serrated strip configuration and includes a plurality of ridges 421, e.g., block-like elements, disposed in a spaced apart relation to one another. Structural support member 426 and the protruding element 422 of the jaw member 420 are similar to the structural support member 126 and the protruding element 122, respectively, of the second jaw member 120 of FIGS. 3 and 5, except for the serrated strip configuration of the protruding element 422, and further description thereof is omitted in the interests of brevity. The shape and size of the ridges 421 may be varied from the configuration depicted in FIGS. 4A and 4B.

In FIG. 6, a curved configuration of an end-effector assembly 600 is shown and includes opposing first and second jaw members 610 and 620. In some embodiments, as shown in FIG. 6, the first jaw member 610 includes an electrically-conductive tissue-engaging surface 612 and the second jaw member 620 includes a protruding element 622, which has a curvilinear configuration. A vibration coupler 660 is configured to impart mechanical vibration to the second jaw member 620 to treat tissue (e.g., tissue "T" shown in FIG. 8) positioned within the end-effector assembly 600. End-effector assembly 600 is similar to the end-effector assembly 100 of FIGS. 1, 3 and 5, except for the shape of the jaw members 610 and 620 and the curvilinear configuration of the protruding element 622, and further description thereof is omitted in the interests of brevity.

FIG. 7 shows an end-effector assembly 700 that includes a first jaw member 710 and the second jaw member 120 of the end-effector assembly 100 (see FIG. 3) disposed in opposing relation relative to one another. First jaw member 710 includes a first structural support member 716 and a protruding element 712 extending from a tissue-engaging surface 729 associated with the first structural support member 716. In some embodiments, the tissue-engaging surface 729 may be a single face of the first structural support member 716. In some embodiments, the conductive sealing surface 129 (and/or the protruding element 714 associated with the first jaw member 711) may be configured to be electrically connectable to the monopolar active terminal of an electrosurgical generator. In examples not part of the present invention, the sealing surface 129 may be formed of an electrically non-conductive material, and the protruding element 122 and/or the protruding element 714 may be configured to be electrically connectable to the monopolar active terminal of an electrosurgical generator.

In some embodiments, as shown in FIG. 7, the element 712 is configured as an elongated strip extending along a length of the first jaw member 710. Alternatively, the protruding element 712 may be configured as an elongated ribbon-like member, a wire, a wire-like member, a rod-like member, or a deposited coating. In some embodiments, the protruding element 712 may be formed of an electrically-conductive material, e.g., metal, on the tissue-engaging surface 729 (and/or first structural support member 716), e.g., using a deposition technique, stamping, etching, machining, and/or any other suitable method that may be used to form an elongated strip-like conductor. In other embodiments, the protruding element 712 may be formed of an electrically non-conductive material.

In alternative configurations, the end-effector assembly 700 may include an electrically-conductive protruding element configured as an elongated strip extending along a length of one jaw member and an electrically non-conductive protruding element configured as an elongated strip extending along a length of the other jaw member. In any of the end-effector assembly embodiments described in this description, the electrically-conductive protruding element (and/or electrically non-conductive protruding element) may include a plurality of serrations.

End-effector assembly 700 may include one or more electrically-insulative elements to electrically isolate the first jaw member 710 from the second jaw member 120. In some embodiments, the protruding element 712 associated with the first jaw member 710 (and/or the protruding element 122 associated with the second jaw member 120) may have a thickness that varies from a proximal end to a distal end thereof. For example, protruding elements 712 and 122 (also referred to herein as "first and second protruding elements 712 and 122") each may have a proximal end having a thickness that is slightly larger than a thickness at the distal end thereof, e.g., depending on a particular purpose. End-effector assembly 700 may additionally, or alternatively, include electrically-insulative members and/or electrically-insulative, thermally non-degrading coatings configured to electrically isolate, at least in part, the first and second protruding elements 712 and 122 from the first and second structural support members 716 and 126, respectively.

In other embodiments, when the first and second jaw members 710 and 120 are disposed in an unapproximated (open) configuration (FIG. 1), upon activation, monopolar electrosurgical energy is provided to one of the first and second protruding elements 712 and 122. In some embodiments, when the first and second jaw members 710 and 120 are disposed in a closed configuration and/or a clamping configuration, upon activation, if it is determined that tissue (e.g., tissue "T" shown in FIG. 8) is disposed between the first and second jaw members 710 and 120, while monopolar electrosurgical energy is provided to one or both of the first and second protruding elements 712 and 122, one of the protruding elements (e.g., second protruding element 122) is employed to provide a mechanical cutting action on tissue "T" by providing a mechanical vibration to the second jaw member 120.

In other embodiments, when the first and second jaw members 710 and 120 are disposed in a closed configuration and/or a clamping configuration, upon activation, if it is determined that tissue "T" is disposed between the first and second jaw members 710 and 120, while electrosurgical energy is provided to the tissue-engaging surfaces 729 and 129, the second protruding element 122 is employed to provide a mechanical cutting action on tissue "T" by providing a mechanical vibration to the second jaw member 120.

In FIG. 8, the end-effector assembly 100 of FIG. 1 is shown disposed in a closed configuration wherein the jaw members 110 and 120 cooperate to grasp tissue "T" therebetween. The thickness of tissue "T" being grasped may be controlled based on the gap distance "G" between the jaw members 110 and 120. In some embodiments, the gap distance "G" is within the range of about 0.001 inches (about 0.025 millimeters) to about 0.015 inches (about 0.381 millimeters). In some embodiments, the gap distance "G" is used as a sensed feedback to control the jaw closure rate and/or thickness of the tissue "T" being grasped. End-effector assembly 100 may include a pair of opposing sensors (not shown) configured to provide real-time feedback relating to the gap distance or closing pressure between the jaw members 110 and 120 during the sealing process. In some embodiments, the protruding element 122 may be configured to determine the gap distance "G." In some embodiments, the end-effector assembly 101 may include a jaw sensing system that detects and/or confirms jaw closure about tissue and/or detects the relative angle of two opposing jaw members relative to one another. Examples of jaw member and sensor configurations of jaw angle detection systems for an end-effector assembly are disclosed in U.S. Patent No. 8,357,158 entitled "Jaw Closure Detection System,"

FIG. 9 shows a schematic block diagram of the electrosurgical power generating source 28 of FIG. 1 including a controller 920, a power supply 927, an RF output stage 928, and a sensor module 922. In some embodiments, as shown in FIG. 9, the sensor module 922 is formed integrally with the electrosurgical power generating source 28. In other embodiments, the sensor module 922 may be provided as separate circuitry coupled to the electrosurgical power generating source 28. The power supply 927 provides DC power to the RF output stage 928 which then converts the DC power into RF energy and delivers the RF energy to the instrument 10 (FIG. 1). The controller 920 includes a microprocessor 925 having a memory 926 which may be volatile type memory (e.g., RAM) and/or non-volatile type memory (e.g., flash media, disk media, etc.). The microprocessor 925 includes an output port connected to the power supply 927 and/or RF output stage 928 that allows the microprocessor 925 to control the output of the generator 28 according to either open and/or closed control loop schemes.

A closed loop control scheme generally includes a feedback control loop wherein the sensor module 922 provides feedback to the controller 920 (e.g., information obtained from one or more sensing mechanisms for sensing various tissue parameters such as tissue impedance, tissue temperature, output current and/or voltage, etc.). The controller 920 then signals the power supply 927 and/or RF output stage 928 which then adjusts the DC and/or RF power supply, respectively. The controller 920 also receives input signals from the input controls of the electrosurgical power generating source 28 and/or instrument 10 (FIG. 1). The controller 920 utilizes the input signals to adjust one or more operating parameters associated with the electrosurgical power generating source 28 and/or instructs the electrosurgical power generating source 28 to perform other control functions.

In some embodiments, the controller 920 is configured to cause the oscillation mechanism 30 to be energized automatically by the electrosurgical power generating source 28 based on one or more signals indicative of conditions and/or operational parameters, e.g., duration of application of RF energy, tissue impedance, temperature, mode of operation, power, current, voltage, first jaw member 110 opening angle, force applied to tissue, and/or thickness of tissue.

The microprocessor 925 is capable of executing software instructions for processing data received by the sensor module 922, and for outputting control signals to the electrosurgical power generating source 28, accordingly. The software instructions, which are executable by the controller 920, are stored in the memory 926 of the controller 920.

The controller 920 may include analog and/or logic circuitry for processing the sensed values and determining the control signals that are sent to the electrosurgical power generating source 28, rather than, or in combination with, the microprocessor 925. The sensor module 922 may include a plurality of sensors (not shown) strategically located for sensing various properties or conditions, e.g., tissue impedance, voltage at the tissue site, current at the tissue site, etc. The sensors are provided with leads (or wireless) for transmitting information to the controller 920. The sensor module 922 may include control circuitry that receives information from multiple sensors, and provides the information and the source of the information (e.g., the particular sensor providing the information) to the controller 920.

FIG. 10 shows a first oscillation mechanism 1000 (also referred to herein as "piezoelectric oscillation mechanism 1000") that includes a piezoelectric device 1014 and a fulcrum 1016. Piezoelectric device 1014 and the fulcrum 1016 are coupled between a base 1012 and an armature 1010. Piezoelectric oscillation mechanism 1000 is configured to be coupled via the vibration coupler 60 to the second jaw member 120 of the end-effector assembly 100 of the electrosurgical instrument 10 of FIG. 1.

In FIG. 11 a second oscillation mechanism 1100 (also referred to herein as "eccentric cam oscillation mechanism 1100") is shown and includes an eccentric 1110 and an electric motor 1120. Eccentric cam oscillation mechanism 1100 is configured to be coupled via the vibration coupler 60 to the second jaw member 120 of the end-effector assembly 100 of the electrosurgical instrument 10 of FIG. 1.

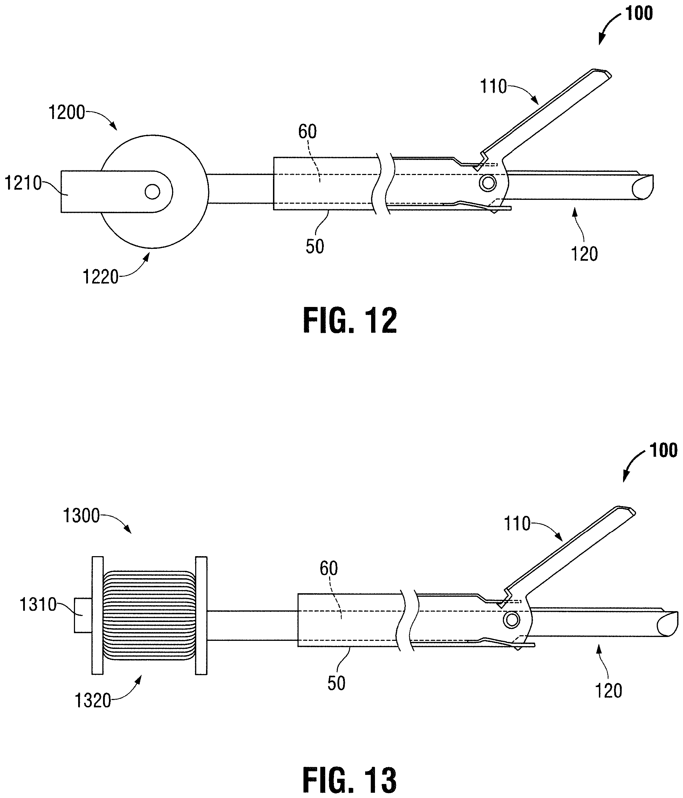

FIG. 12 shows a third oscillation mechanism 1200 (also referred to herein as "counterweight oscillation mechanism 1200") that includes an electric motor 1220 and a counterweight 1210. Counterweight oscillation mechanism 1200 is configured to be coupled via the vibration coupler 60 to the second jaw member 120 of the end-effector assembly 100 of the electrosurgical instrument 10 of FIG. 1.

FIG. 13 shows a fourth oscillation mechanism 1300 (also referred to herein as "voice coil oscillation mechanism 1300") that includes a magnetic element 1310 and a coil element 1320. Voice coil oscillation mechanism 1300 is configured to be coupled via the vibration coupler 60 to the second jaw member 120 of the end-effector assembly 100 of the electrosurgical instrument 10 of FIG. 1.

In some variations of vibration couplers, compatible with any of the jaw member embodiments disclosed herein, oscillation mechanisms 1000, 1100, 1200, or 1300 shown in FIGS. 10 through 13, respectively, may be operably coupled to one of the opposing jaw members of the presently-disclosed end-effector assemblies (e.g., the end-effector assembly 600 shown in FIG. 6 or the end-effector assembly 701 shown in FIG. 7).

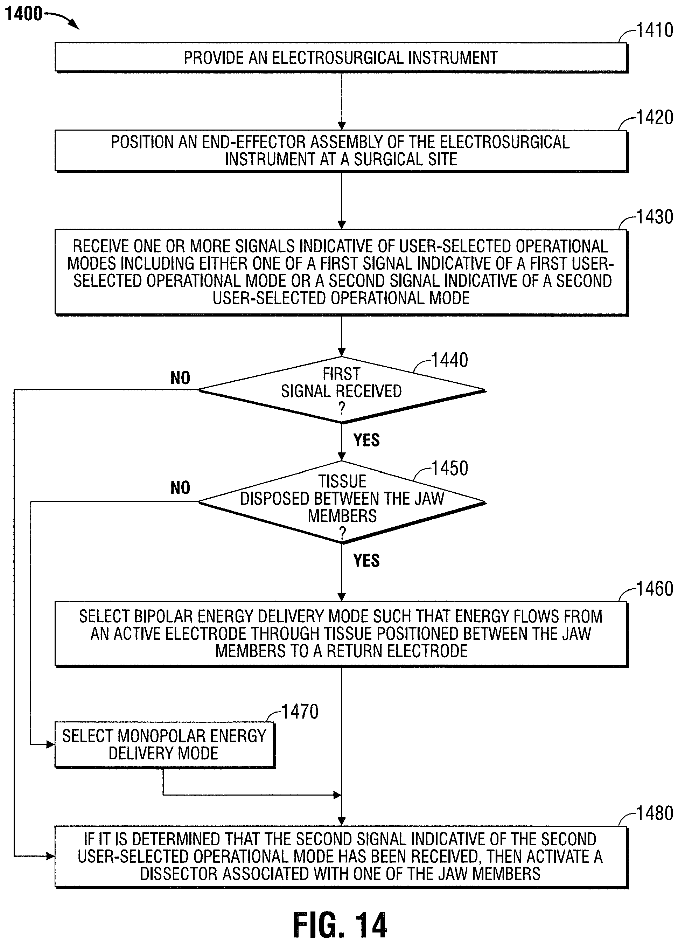

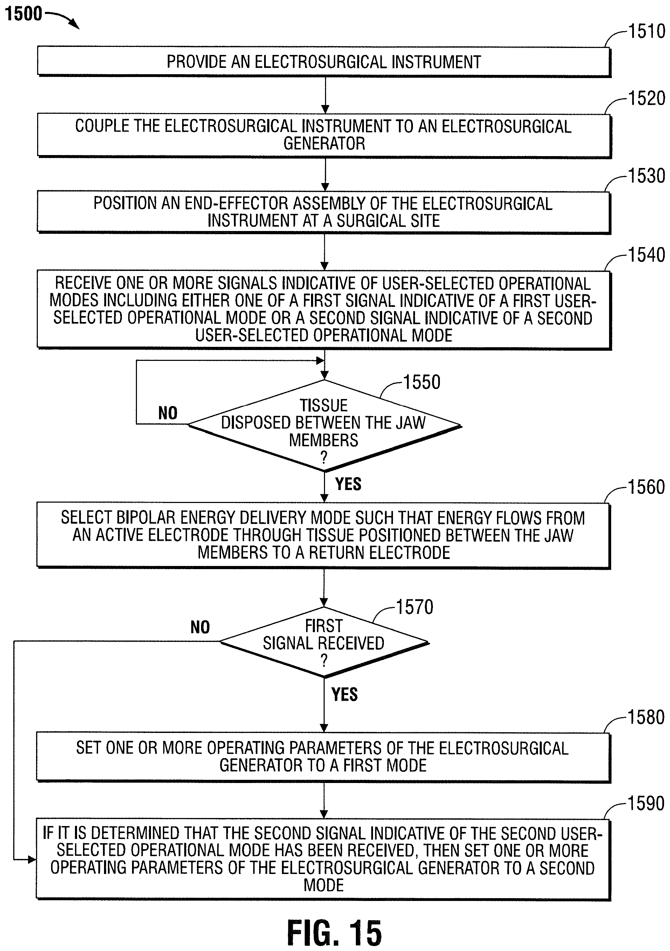

Hereinafter, exemplary methods of treating tissue, in accordance with the present disclosure, are described with reference to FIGS. 14 and 15. It is to be understood that the steps of the methods provided herein may be performed in combination and in a different order than presented herein without departing from the scope of the disclosure.

FIG. 14 is a flowchart illustrating a method of treating tissue. In step 1410, an electrosurgical instrument 10 is provided. The instrument 10 includes an elongated shaft 50 having an end-effector assembly 700 at a distal end thereof. The end-effector assembly 700 assembly includes opposing jaw members 710 and 120, at least one of which is movable from a first position wherein the jaw members 710 and 120 are disposed in spaced relation relative to one another to at least a second position closer to one another wherein the jaw members 710 and 120 cooperate to grasp tissue "T" therebetween.

Jaw members 710 and 120 each include a tissue-engaging surface and 729 and 129, respectively, and a protruding element 712 and 122, respectively extending therefrom. The electrically-conductive protruding element 712 may be configured as a wire or a rod-like member, or as an elongated ribbon-like or strip-like member, or as a deposited coating. One or both of the protruding elements 712 and 122 is made of an electrically-conductive material.

In some embodiments, the protruding elements 712 and 122 are configured to determine a gap distance "G" between the jaw members 710 and 120. Electrosurgical instrument 10 is configured to generate one or more signals indicative of user-selected operational modes.

In step 1420, the end-effector assembly 700 is positioned at a surgical site.

In step 1430, one or more signals indicative of user-selected operational modes are received, including a first signal indicative of a first user-selected operational mode and/or a second signal indicative of a second user-selected operational mode.

In step 1440, a determination is made whether the first signal indicative of the first user-selected operational mode has been received. If it is determined that the first signal indicative of the first user-selected operational mode has been received, in step 1440, then a determination is made whether tissue "T" is disposed between the jaw members 710 and 120 in step 1450. If it is determined that the first signal indicative of the first user-selected operational mode has not been received, in step 1440, then a determination is made whether the second signal indicative of the second user-selected operational mode has been received in step 1480.

In step 1450, a determination is made whether tissue "T" is disposed between the jaw members 710 and 120. If it is determined that tissue "T" is disposed between the jaw members 710 and 120, in step 1450, then a bipolar energy delivery mode is selected in step 1460. In bipolar energy delivery mode, the protruding element 712, made of an electrically-conductive material, functions as an active electrode or a return electrode during activation. If it is determined that tissue "T" is not disposed between the jaw members 710 and 120, in step 1450, then a monopolar energy delivery mode is selected in step 1470.

In step 1480, if it is determined that the second signal indicative of the second user-selected operational mode has been received, then oscillating mechanical energy is provided to one of the jaw members 120.

FIG. 15 is a flowchart illustrating a method of treating tissue according to an embodiment of the present disclosure. In step 1510, an electrosurgical instrument 10 is provided. The instrument 10 includes an end-effector assembly 100 having opposing jaw members 110 and 120. Each of the jaw members 110 and 120 includes an electrically-conductive element. One of the jaw members (e.g., jaw member 110) is movable relative to the other (e.g., jaw member 120) from a first position wherein the jaw members 110 and 120 are disposed in spaced relation relative to one another to at least a second position closer to one another wherein the jaw members 110 and 120 cooperate to grasp tissue "T" therebetween. Jaw members 110 and 120 include electrically-conductive elements 112 and 129, respectively, each connectable to an energy source 28. Electrosurgical instrument 10 is configured to generate one or more signals indicative of user-selected operational modes.

In step 1520, the electrosurgical instrument 10 is coupled to an electrosurgical generator 28. Electrosurgical generator 28 may be any generator suitable for use with electrosurgical devices, and may be configured to operate in a variety of modes such as monopolar and bipolar cutting, coagulation, and other modes.

In step 1530, the end-effector assembly 100 is positioned at a surgical site.

In step 1540, one or more signals indicative of user-selected operational modes are received, e.g., a first signal indicative of a first user-selected operational mode or a second signal indicative of a second user-selected operational mode.

In step 1550, a determination is made whether tissue "T" is disposed between the jaw members 110 and 120. If it is determined that tissue "T" is disposed between the jaw members 110 and 120, in step 1550, then a bipolar energy delivery mode is selected in step 1560. In bipolar energy delivery mode, one of the electrically-conductive elements, e.g., 112, functions as an active electrode and the other electrically-conductive element, e.g., 129, functions as a return electrode during activation such that energy flows from the active electrode through tissue positioned between the electrically-conductive elements 112 and 129 to the return electrode.

In step 1570, a determination is made whether the first signal indicative of the first user-selected operational mode has been received. If it is determined that the first signal indicative of the first user-selected operational mode has been received, in step 1570, then one or more operating parameters of the electrosurgical generator 28 are set to a first mode in step 1580. If it is determined that the first signal indicative of the first user-selected operational mode has not been received, in step 1570, then a determination is made whether the second signal indicative of the second user-selected operational mode has been received in step 1590.

Some examples of operating parameters associated with the electrosurgical generator 28 that may be set, e.g., to a first mode or a second mode, or otherwise adjusted, include temperature, impedance, power, current, voltage, mode of operation, and duration of application of electrosurgical energy.

In step 1590, if it is determined that the second signal indicative of the second user-selected operational mode has been received, then one or more operating parameters of the electrosurgical generator 28 are set to a second mode.

The above-described electrosurgical instruments having an end-effector assembly coupled to a vibration mechanism and configured to provide a mechanical cutting action on tissue may be suitable for sealing, cauterizing, coagulating, desiccating, and/or cutting tissue, e.g., vessels and vascular tissue. The above-described electrosurgical instruments configured to provide monopolar electrosurgical energy and/or bipolar electrosurgical energy may be suitable for utilization in endoscopic surgical procedures and/or suitable for utilization in open surgical applications.

One or both of the jaw members of the above-described end-effector assemblies may be provided with a protruding element, which may have a curvilinear configuration, abrasiveness characteristics, and/or serrations. The above-described protruding elements may be configured to extend from a tissue-engaging surface of one or both of the jaw members, and may be configured as an elongated ribbon-like or strip-like member, a wire, a wire-like member, a rod-like member, or a deposited coating. The above-described protruding elements may be configured to function as an oscillating cutting element.

The various embodiments disclosed herein may also be configured to work with robotic surgical systems and what is commonly referred to as "Telesurgery." Such systems employ various robotic elements to assist the surgeon in the operating theatre and allow remote operation (or partial remote operation) of surgical instrumentation. Various robotic arms, gears, cams, pulleys, electric and mechanical motors, etc. may be employed for this purpose and may be designed with a robotic surgical system to assist the surgeon during the course of an operation or treatment. Such robotic systems may include remotely-steerable systems, automatically flexible surgical systems, remotely-flexible surgical systems, remotely-articulating surgical systems, wireless surgical systems, modular or selectively configurable remotely-operated surgical systems, etc.

The robotic surgical systems may be employed with one or more consoles that are next to the operating theater or located in a remote location. In this instance, one team of surgeons or nurses may prep the patient for surgery and configure the robotic surgical system with one or more of the instruments disclosed herein while another surgeon (or group of surgeons) remotely controls the instruments via the robotic surgical system. As can be appreciated, a highly skilled surgeon may perform multiple operations in multiple locations without leaving his/her remote console which can be both economically advantageous and a benefit to the patient or a series of patients.

The robotic arms of the surgical system are typically coupled to a pair of master handles by a controller. The handles can be moved by the surgeon to produce a corresponding movement of the working ends of any type of surgical instrument (e.g., end effectors, graspers, knifes, scissors, etc.) which may complement the use of one or more of the embodiments described herein. The movement of the master handles may be scaled so that the working ends have a corresponding movement that is different, smaller or larger, than the movement performed by the operating hands of the surgeon. The scale factor or gearing ratio may be adjustable so that the operator can control the resolution of the working ends of the surgical instrument(s).

The master handles may include various sensors to provide feedback to the surgeon relating to various tissue parameters or conditions, e.g., tissue resistance due to manipulation, cutting or otherwise treating, pressure by the instrument onto the tissue, tissue temperature, tissue impedance, etc. As can be appreciated, such sensors provide the surgeon with enhanced tactile feedback simulating actual operating conditions. The master handles may also include a variety of different actuators for delicate tissue manipulation or treatment further enhancing the surgeon's ability to mimic actual operating conditions.

## Claims

1. An electrosurgical instrument (10), comprising:
a housing (20);
a shaft (50) coupled to the housing, the shaft having a proximal end and a distal end;
an end-effector assembly (100,600) disposed at the distal end of the shaft, the end-effector assembly including first and second jaw members (110,120,420,610,620), at least one of the first and second jaw members movable from a first position wherein the first and second jaw members are disposed in spaced relation relative to one another to at least a second position closer to one another wherein the first and second jaw members cooperate to grasp tissue therebetween;
a vibration coupler (60) including a distal end movably coupled to a proximal end of the second jaw member, wherein the vibration coupler is configured to impart a mechanical vibration to the second jaw member to treat tissue disposed between the first and second jaw members; and
an oscillation mechanism (30,1000,1100,1200,1300) configured to generate mechanical vibration in the vibration coupler,
wherein:
the first jaw member (110,610) includes an electrically-conductive tissue-engaging surface (112); and
the second jaw member (120,420,620) includes an electrically-conductive tissue-engaging surface (129) and a protruding element (122,422,622) extending therefrom, the protruding element configured as an elongated strip extending along a length of the second jaw member;
**characterized in that** the electrically-conductive tissue-engaging surface (112) of the first jaw member is configured as a tissue-sealing plate facing the electrically-conductive tissue-engaging surface of the second jaw member, and the protruding element (122,422,622) is made of an electrically non-conducting material located centrally within the electrically-conductive tissue-engaging surface (129) of the second jaw member (120,420,620) in opposing relation to a central portion of the electrically-conductive tissue-engaging surface (112) of the first jaw member (110,610).

2. The electrosurgical instrument of claim 1, wherein the first jaw member (710) includes a further protruding element (712) extending therefrom, the further protruding element configured as an elongated strip extending along a length of the first jaw member and being formed of electrically-conductive material.

3. The electrosurgical instrument of any preceding claim, wherein the end effector assembly is configured to provide bipolar electrosurgical energy to tissue disposed between the first and second jaw members.

4. The electrosurgical instrument of any preceding claim, wherein the protruding element (422) on said second jaw member (420) includes serrations.

5. The electrosurgical instrument of any preceding claim, wherein the vibration coupler (60) is integrally formed with the proximal end of the second jaw member (120,420,620).

6. The electrosurgical instrument of any preceding claim, further comprising a two-stage switch (72), a first stage of the two-stage switch effecting a first operational mode of at least two operational modes and a second stage of the two-stage switch effecting a second operational mode of the at least two operational modes that is different from the first operational mode.

7. The electrosurgical instrument of claim 6, wherein the two-stage switch (72) is configured to transition to the first operational mode when a first depression force is applied to the two-stage switch, wherein bipolar electrosurgical energy is provided for sealing.

8. The electrosurgical instrument of claim 6 or 7, wherein the two-stage switch (72) is configured to transition to the second operational mode when a second depression force is applied to the two-stage switch, wherein the mechanical vibration is imparted to the second jaw member.

9. The electrosurgical instrument of any preceding claim, further comprising:
an electrosurgical generator (28);
wherein the electrosurgical instrument is configured to generate one or more signals indicative of user-selected operational modes including either one of a first signal indicative of a first user-selected operational mode or a second signal indicative of a second user-selected operational mode;
means for determining that the first signal indicative of the first user-selected operational mode has been provided, then for selecting a bipolar energy delivery mode, wherein one of the electrically-conductive tissue-engaging surface (112) of the first jaw member (110,610) and the electrically-conductive tissue-engaging surface (129) of the second jaw member (120,420,620) functions as an active electrode and the other one of the electrically-conductive tissue-engaging surface of the first jaw member and the electrically-conductive tissue-engaging surface of the second jaw member functions as a return electrode during activation such that energy flows from the active electrode through tissue positioned between the electrically-conductive elements to the return electrode, and
means for determining that the second signal indicative of the second user-selected operational mode has been provided, then for setting a mechanical energy delivery mode, wherein oscillating mechanical energy is provided to the second jaw member.

10. The electrosurgical instrument of claim 9, wherein the oscillating mechanical energy is configured to divide tissue.

## Patentansprüche

1. Elektrochirurgisches Instrument (10), umfassend:
ein Gehäuse (20);
einen Schaft (50), der mit dem Gehäuse gekoppelt ist, wobei der Schaft ein proximales Ende und ein distales Ende aufweist;
eine Endeffektoranordnung (100, 600), die an dem distalen Ende des Schaftes angeordnet ist, wobei die Endeffektoranordnung erste und zweite Klemmbackenelemente (110, 120, 420, 610, 620) aufweist, wobei zumindest eines der ersten und zweiten Klemmbackenelemente von einer ersten Position, bei der die ersten und zweiten Klemmbackenelemente in einer beabstandeten Beziehung relativ zueinander angeordnet sind, in zumindest eine zweite Position näher aneinander beweglich ist, bei der die ersten und zweiten Klemmbackenelemente zusammenwirken, um Gewebe dazwischen zu ergreifen;
einen Vibrationskoppler (60), der ein distales Ende aufweist, das mit einem proximalen Ende des zweiten Klemmbackenelementes beweglich gekoppelt ist, wobei der Vibrationskoppler konfiguriert ist, um dem zweiten Klemmbackenelement eine mechanische Vibration zu verleihen, um zwischen den ersten und zweiten Klemmbackenelementen angeordnetes Gewebe zu behandeln; und
einen Oszillationsmechanismus (30, 1000, 1100, 1200, 1300), der konfiguriert ist, um mechanische Vibration in dem Vibrationskoppler zu erzeugen,
wobei:
das erste Klemmbackenelement (110, 610) eine elektrisch leitende Gewebeeingriffsfläche (112) aufweist; und
das zweite Klemmbackenelement (120, 420, 620) eine elektrisch leitende Gewebeeingriffsfläche (129) und ein sich davon erstreckendes, vorstehendes Element (122, 422, 622) aufweist, wobei das vorstehende Element als ein länglicher Streifen konfiguriert ist, der sich entlang einer Länge des zweiten Klemmbackenelementes erstreckt;
**dadurch gekennzeichnet, dass** die elektrisch leitende Gewebeeingriffsfläche (112) des ersten Klemmbackenelementes als eine Gewebeabdichtungsplatte konfiguriert ist, die der elektrisch leitenden Gewebeeingriffsfläche des zweiten Klemmbackenelementes gegenüberliegt, und das vorstehende Element (122, 422, 622) aus einem elektrisch nicht leitenden Material hergestellt ist, das sich zentral innerhalb der elektrisch leitenden Gewebeeingriffsfläche (129) des zweiten Klemmbackenelementes (120, 420, 620) in gegenüberliegender Beziehung zu einem zentralen Abschnitt der elektrisch leitenden Gewebeeingriffsfläche (120) des ersten Klemmbackenelementes (110, 610) befindet.

2. Elektrochirurgisches Instrument nach Anspruch 1, wobei das erste Klemmbackenelement (710) ein weiteres vorstehendes Element (712) aufweist, das sich davon erstreckt, wobei das weitere vorstehende Element als ein länglicher Streifen konfiguriert ist, der sich entlang einer Länge des ersten Klemmbackenelementes erstreckt und aus elektrisch leitendem Material gebildet ist.

3. Elektrochirurgisches Instrument nach einem vorstehenden Anspruch, wobei die Endeffektoranordnung konfiguriert ist, um bipolare elektrochirurgische Energie an zwischen den ersten und zweiten Klemmbackenelementen angeordnetes Gewebe bereitzustellen.

4. Elektrochirurgisches Instrument nach einem vorstehenden Anspruch, wobei das vorstehende Element (422) an dem zweiten Klemmbackenelement (420) Verzahnungen aufweist.

5. Elektrochirurgisches Instrument nach einem vorstehenden Anspruch, wobei der Vibrationskoppler (60) mit dem proximalen Ende des zweiten Klemmbackenelementes (120, 420, 620) einstückig ausgebildet ist.

6. Elektrochirurgisches Instrument nach einem vorstehenden Anspruch, weiter umfassend einen zweistufigen Schalter (72), wobei eine erste Stufe des zweistufigen Schalters einen ersten Betriebsmodus von zumindest zwei Betriebsmodi bewirkt und eine zweite Stufe des zweistufigen Schalters einen zweiten Betriebsmodus von den zumindest zwei Betriebsmodi bewirkt, der sich von dem ersten Betriebsmodus unterscheidet.

7. Elektrochirurgisches Instrument nach Anspruch 6, wobei der zweistufige Schalter (72) konfiguriert ist, um in den ersten Betriebsmodus überzugehen, wenn eine erste Druckkraft auf den zweistufigen Schalter ausgeübt wird, wobei bipolare elektrochirurgische Energie zur Abdichtung bereitgestellt wird.

8. Elektrochirurgisches Instrument nach Anspruch 6 oder 7, wobei der zweistufige Schalter (72) konfiguriert ist, um in den zweiten Betriebsmodus überzugehen, wenn eine zweite Druckkraft auf den zweistufigen Schalter ausgeübt wird, wobei dem zweiten Klemmbackenelement die mechanische Vibration verliehen wird.

9. Elektrochirurgisches Instrument nach einem vorstehenden Anspruch, weiter umfassend:
einen elektrochirurgischen Generator (28);
wobei das elektrochirurgische Instrument konfiguriert ist, um ein oder mehrere Signale zu erzeugen, die auf benutzerausgewählte Betriebsmodi hindeuten, die eines von einem ersten Signal, das auf einen ersten benutzerausgewählten Betriebsmodus hindeutet, und einem zweiten Signal aufweisen, das auf einen zweiten benutzerausgewählten Betriebsmodus hindeutet;
Mittel zum Bestimmen, dass das erste Signal, das auf den ersten benutzerausgewählten Betriebsmodus hindeutet, bereitgestellt wurde, dann zum Auswählen eines bipolare Energie-Lieferungsmodus, wobei eine von der elektrisch leitenden Gewebeeingriffsfläche (112) des ersten Klemmbackenelementes (110, 610) und der elektrisch leitenden Gewebeeingriffsfläche (129) des zweiten Klemmbackenelementes (120, 420, 620) als eine aktive Elektrode fungiert und die andere von der elektrisch leitenden Gewebeeingriffsfläche des ersten Klemmbackenelementes und der elektrisch leitenden Gewebeeingriffsfläche des zweiten Klemmbackenelementes als eine Rückführelektrode fungiert, während einer Aktivierung, so dass Energie von der aktiven Elektrode durch zwischen den elektrisch leitenden Elementen positioniertes Gewebe zur Rückführelektrode fließt, und
Mittel zum Bestimmen, dass das zweite Signal, das auf den zweiten benutzerausgewählten Betriebsmodus hindeutet, bereitgestellt wurde, dann zum Einstellen eines mechanische Energie-Lieferungsmodus, wobei oszillierende, mechanische Energie an das zweite Klemmbackenelement bereitgestellt wird.

10. Elektrochirurgisches Instrument nach Anspruch 9, wobei die oszillierende, mechanische Energie konfiguriert ist, um Gewebe zu teilen.

## Revendications

1. Instrument électrochirurgical (10), comprenant :
un logement (20);
un arbre (50) couplé au logement, l'arbre ayant une extrémité proximale et une extrémité distale ;
un ensemble effecteur terminal (100, 600) disposé à l'extrémité distale de l'arbre, l'ensemble effecteur terminal comprenant des premier et second éléments de mâchoire (110, 120, 420, 610, 620), au moins l'un des premier et second éléments de mâchoire pouvant être déplacé depuis une première position dans laquelle les premier et second éléments de mâchoire sont disposés en relation espacée les uns par rapport aux autres jusqu'à au moins une seconde position plus proche les uns des autres dans laquelle les premier et second éléments de mâchoire coopèrent pour saisir du tissu entre eux ;
un coupleur à vibrations (60) comprenant une extrémité distale couplée de manière mobile à une extrémité proximale du second élément de mâchoire, dans lequel le coupleur à vibrations est configuré pour transmettre une vibration mécanique au second élément de mâchoire pour traiter du tissu disposé entre les premier et second éléments de mâchoire ; et
un mécanisme d'oscillation (30, 1000, 1100, 1200, 1300) configuré pour générer des vibrations mécaniques dans le coupleur à vibrations,
dans lequel :
le premier élément de mâchoire (110, 610) comprend une surface d'engagement de tissu électriquement conductrice (112) ; et
le second élément de mâchoire (120, 420, 620) comprend une surface d'engagement de tissu électriquement conductrice (129) et un élément saillant (122, 422, 622) s'étendant à partir de celle-ci, l'élément saillant étant configuré comme une bande allongée s'étendant sur une longueur du second élément de mâchoire ;
**caractérisé en ce que** la surface d'engagement de tissu électriquement conductrice (112) du premier élément de mâchoire est configurée comme une plaque d'étanchéité de tissu tournée vers la surface d'engagement de tissu électriquement conductrice du second élément de mâchoire, et l'élément saillant (122, 422, 622) est réalisé en un matériau électriquement non conducteur situé de manière centrale à l'intérieur de la surface d'engagement de tissu électriquement conductrice (129) du second élément de mâchoire (120, 420, 620) en relation opposée à une portion centrale de la surface d'engagement de tissu électriquement conductrice (112) du premier élément de mâchoire (110, 610).

2. Instrument électrochirurgical selon la revendication 1, dans lequel le premier élément de mâchoire (710) comprend un autre élément saillant (712) s'étendant à partir de celui-ci, l'autre élément saillant étant configuré comme une bande allongée s'étendant sur une longueur du premier élément de mâchoire et étant formé en un matériau électriquement conducteur.

3. Instrument électrochirurgical selon l'une quelconque des revendications précédentes, dans lequel l'ensemble effecteur terminal est configuré pour fournir de l'énergie électrochirurgicale bipolaire à du tissu disposé entre les premier et second éléments de mâchoire.

4. Instrument électrochirurgical selon l'une quelconque des revendications précédentes, dans lequel l'élément saillant (422) sur ledit second élément de mâchoire (420) comprend des dentelures.

5. Instrument électrochirurgical selon l'une quelconque des revendications précédentes, dans lequel le coupleur à vibrations (60) est formé d'un seul tenant avec l'extrémité proximale du second élément de mâchoire (120, 420, 620).

6. Instrument électrochirurgical selon l'une quelconque des revendications précédentes, comprenant en outre un commutateur à deux positions (72), une première position du commutateur à deux positions actionnant un premier mode opérationnel parmi au moins deux modes opérationnels et une seconde position du commutateur à deux positions actionnant un second mode opérationnel parmi les au moins deux modes opérationnels qui est différent du premier mode opérationnel.

7. Instrument électrochirurgical selon la revendication 6, dans lequel le commutateur à deux positions (72) est configuré pour passer au premier mode opérationnel lorsqu'une première force d'enfoncement est appliquée au commutateur à deux positions, dans lequel de l'énergie électrochirurgicale bipolaire est fournie pour assurer l'étanchéité.

8. Instrument électrochirurgical selon la revendication 6 ou 7, dans lequel le commutateur à deux positions (72) est configuré pour passer au second mode opérationnel lorsqu'une seconde force d'enfoncement est appliquée au commutateur à deux positions, dans lequel la vibration mécanique est transmise au second élément de mâchoire.

9. Instrument électrochirurgical selon l'une quelconque des revendications précédentes, comprenant en outre :
un générateur électrochirurgical (28) ;
dans lequel l'instrument électrochirurgical est configuré pour générer un ou plusieurs signaux indicatifs de modes opérationnels sélectionnés par l'utilisateur comprenant l'un quelconque parmi un premier signal indicatif d'un premier mode opérationnel sélectionné par l'utilisateur ou un second signal indicatif d'un second mode opérationnel sélectionné par l'utilisateur ;
des moyens pour déterminer que le premier signal indicatif du premier mode opérationnel sélectionné par l'utilisateur a été fourni, puis pour sélectionner un mode de délivrance d'énergie bipolaire, dans lequel l'une parmi la surface d'engagement de tissu électriquement conductrice (112) du premier élément de mâchoire (110, 610) et la surface d'engagement de tissu électriquement conductrice (129) du second élément de mâchoire (120, 420, 620) sert d'électrode active et l'autre parmi la surface d'engagement de tissu électriquement conductrice du premier élément de mâchoire et la surface d'engagement de tissu électriquement conductrice du second élément de mâchoire sert d'électrode de référence pendant l'activation de telle sorte que de l'énergie circule de l'électrode active à travers le tissu positionné entre les éléments électriquement conducteurs, jusqu'à l'électrode de référence, et
des moyens pour déterminer que le second signal indicatif du second mode opérationnel sélectionné par l'utilisateur a été fourni, puis pour régler un mode de fourniture d'énergie mécanique, dans lequel de l'énergie mécanique oscillante est fournie au second élément de mâchoire.

10. Instrument électrochirurgical selon la revendication 9, dans lequel l'énergie mécanique oscillante est configurée pour diviser du tissu.
